(19) Europäisches Patentamt
European Patent Office
Office européen des brevets

(11) Publication number: 0 131 839 B1

# (12) EUROPEAN PATENT SPECIFICATION

(45) Date of publication of patent specification: 01.02.89

(21) Application number: 84107688.8

(22) Date of filing: 03.07.84

(51) Int. Cl.$^4$: C 07 D 215/56, C 07 D 401/04, C 07 D 405/04, C 07 D 409/04, C 07 D 417/04, C 07 D 405/14, C 07 D 401/14, A 61 K 31/47

## (54) Quinoline antibacterial compounds.

(30) Priority: 18.07.83 US 514716
26.01.84 US 574227
09.04.84 US 597854

(43) Date of publication of application:
23.01.85 Bulletin 85/04

(45) Publication of the grant of the patent:
01.02.89 Bulletin 89/05

(84) Designated Contracting States:
AT BE CH DE FR GB IT LI LU NL SE

(56) References cited:
EP-A-0 045 375
DE-A-2 840 910
DE-A-3 205 655
GB-A-1 147 336
US-A-4 017 622

CHEMICAL ABSTRACTS, vol. 96, no. 23, June 7, 1982, Columbus, Ohio, USA NAGATSU, Y; IRIKURA, T. "Synthesis of carbon-14-labeled antibacterial agent. Synthesis of 1-ethyl-(1-14C)-6-fluoro-1,4-dihydro-4-oxo-7-(1-piperazinyl)-3-quinoline-carboxylic acid (14 C-AM-715)" page 669, column 1, abstract-no. 199 632t

CHEMICAL ABSTRACTS, vol. 96, no. 21, May 24, 1982, Columbus, Ohio, USA KYORIN PHARMACEUTICAL CO., LTD. "Quinolinecarboxylic acid derivatives" page 717, column 1, abstract-no. 181 311c

The file contains technical information submitted after the application was filed and not included in this specification

(73) Proprietor: ABBOTT LABORATORIES
14th Street and Sheridan Road North St
North Chicago Illinois 60064 (US)

(72) Inventor: Chu, Daniel Tim-Wo
204 Ashland Court
Vernon Hills Illinois (US)

(74) Representative: Modiano, Guido et al
MODIANO, JOSIF, PISANTY & STAUB Modiano & Associati Via Meravigli, 16
I-20123 Milan (IT)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 131 839 B1

Courier Press, Leamington Spa, England.

(56) References cited:

CHEMICAL ABSTRACTS, vol. 96, no. 13, March 29, 1982, Columbus, Ohio, USA IRIKURA, TSUTOMU; SUZUKI, HIROSHI; SUGIMOTO, TSUTOMU "Reproduction studies of AM-715 in mice. I. Fertility study" page 38, column 1, abstract-no. 97 222s

CHEMICAL ABSTRACTS, vol. 96, no. 13, March 29, 1982, Columbus, Ohio, USA IRIKURA, TSUTOMU; SUZUKI, HIROSHI; SUGIMOTO; TSUTOMU "Mutagenicity studies of AM-715 in animals" page 38, column 2, abstract-no. 97 225v

CHEMICAL ABSTRACTS, vol. 96, no. 13, March 29, 1982, Columbus, Ohio, USA ABIKO, TOYO; ISHIHAMA, ATSUSHI; OGAWA, NOBUYA; UCHIDA, HIROSHI: MURAYAMA, SATOSHI; HIRAI, KEIJI; OOMORI, YASUO; ABE, YASUO; IRIKURA, TSUTOMU "Phase I study on AM-715" page 21, column 1, abstract-no. 97 091y

CHEMICAL ABSTRACTS, vol. 97, no. 15, October 15, 1982, Columbus, Ohio, USA NEWSOM, S.W.B., MATTHEWS, JULIE; AMPHLETT, M.; WARREN, R.E. "Norfloxacin and the antibacterial gamma pyridone beta carboxylic acids" page 380, column 2, abstract-no. 123 794x

CHEMICAL ABSTRACTS, vol. 98, no. 15, April 11, 1983, Columbus, Ohio, USA KYORIN PHARMACEUTICAL CO., LTD. "Pyridonecarboxylic acid derivatives" page 623, column 1, abstract-no. 125 905d

CHEMICAL ABSTRACTS, vol. 98, no. 13, March 28, 1983, Columbus, Ohio, USA TANI, JUNICHI; MUSHIKA, YOSHITAKA; YAMAGUCHI, TOTARO "Studies on biologically active halogenated compounds. IV. Synthesis and antibacterial activity of fluorinated quinoline derivatives" page 587, column 1, abstract-no. 107 135y

**Description**

This invention relates to new and useful quinoline derivatives having antibacterial properties, to compositions containing the new quinoline derivatives and these new quinoline derivatives for use in methods of treating mammalian patients.

It is known that certain 7-piperazinyl-4-oxo-1,4-dihydroquinoline-3-carboxylic acids exhibit antibacterial properties. For example, U.S. Patent No. 4,017,622 discloses certain 7-piperazinyl-4-oxo-1,4-dihydroquinoline-3-carboxylic acid derivatives wherein the 1 position substituent is alkyl, benzyl or acetyl. U.S. Patent No. 4,292,317 discloses certain 7-piperazinyl-6-4-halo-oxo-1,4-dihydroquinoline-3-carboxylic acid derivatives wherein the the 1 position substituent is methyl, ethyl, vinyl or alkyl. In U.S. Patent No. 4,284,629, various 4-oxo-1,4-dihydroquinoline-3-carboxylic acids are disclosed in which the 1 position substituent may be cycloalkyl, although corresponding derivatives containing a 7-piperazinyl substituent are not disclosed. From GB—A—1 147 336 (substituted) 1-aryl-1,4-dihydro-4-oxo-quinoline-3-carboxylic acid derivatives are known having antibacterial activity. While the compounds of the foregoing patents may be useful in certain respects, the search continues for new quinoline derivatives which have improved properties or are otherwise useful in the treatment of bacterial infections.

The present invention relates to new 7-substituted 6-fluoro-1,4-dihydro-4-oxo-quinoline-3-carboxylic acids or esters having a substituted or unsubstituted phenyl radical or an aromatic heterocyclic radical in the 1 position, to compositions of the new compounds together with pharmaceutically acceptable carriers, and to uses of the new compounds in the treatment of bacterial infections.

The compounds of the invention can be represented by the following Formula I:

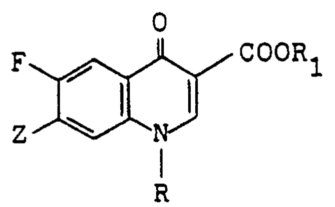

wherein $R_1$ is hydrogen or a carboxy protecting group; R is a phenyl group having the formula:

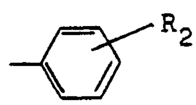

wherein $R_2$ is one or more groups selected from the group consisting of hydrogen, halogen, methylenedioxy, $C_1$ to $C_6$ alkyl, and a group having the formula:

$$-OR_3$$

wherein $R_3$ is hydrogen or $C_1$ to $C_6$ alkyl; and Z is a heterocyclic ring having the structure:

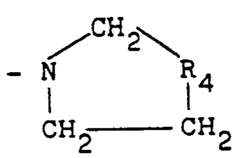

wherein $R_4$ is $CH_2$, $(CH_2)_2$ or $(CH_2)_nNH$— wherein n is 1 or 2 and substituted derivatives thereof, wherein said substituents are selected from one or more of $C_1$ to $C_6$ alkyl and $—NR_5R_6$ wherein $R_5$ and $R_6$ are independently selected from hydrogen, $C_1$ to $C_6$ alkyl and alkanoyl; and pharmaceutically acceptable salts thereof.

As used herein, the term "halogen" refers to chloro, bromo, fluoro, and iodo groups, while the term "$C_1$ to $C_4$ alkyl" refers to lower alkyl groups including methyl, ethyl, propyl, isopropyl, butyl.

As indicated above, $R_2$ can be amongst other straight or branched chain $C_1$ to $C_6$ alkyl or a group of the formula $—O—R_3$. Representative groups of this type include a hydroxy group and a lower alkoxy group, such as methoxy, ethoxy, propoxy, etc.

As used herein, the term "carboxy-protecting group" refers to and includes the residue of a carboxylic acid ester group. Such carboxy-protecting groups are well known to those skilled in the art, having been extensively used in the protection of carboxyl groups in the penicillin and cephalosporin fields, as described in U.S. Patent Nos. 3,840,556 and 3,719,667, the disclosures of which are incorporated herein by

reference. In general, such carboxy-protecting groups can be relatively easily cleaved to yield the corresponding free carboxy group.

The heterocyclic rings representing Z are, in accordance with the preferred practice of the invention, aliphatic (saturated) heterocyclic rings containing 1 or 2 N atoms. Also included are substituted derivatives of such heterocyclic rings wherein the substituent is one or more of a $C_1$ to $C_4$ alkyl group, an amine group having the formula:

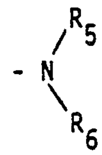

wherein $R_5$ and $R_6$ are each independently selected from the group consisting of hydrogen, $C_1$ to $C_4$ alkyl and alkanoyl.

Illustrative of the heterocyclic groups representing Z are azetidinyl groups, piperazinyl groups, piperidinyl groups, pyrrolidinyl groups.

The preferred compounds of the present invention are those having the formula:

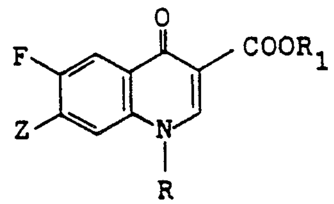

wherein R is phenyl or substituted phenyl wherein the substituent on the phenyl group is one or more of alkyl, halogen, methylenedioxy, or hydroxy, $R_1$ is as described above, preferably hydrogen, and Z is piperazinyl, substituted piperazinyl or substituted pyrrolidinyl as described above.

Representative of such preferred compounds include 1 - phenyl - 6 - fluoro - 1,4 - dihydro - 4 - oxo - 7 - (1 - piperazinyl) - quinoline - 3 - carboxylic acid, 1 - phenyl - 6 - fluoro - 1,4 - dihydro - 4 - oxo - 7 - (1 - (4 - methyl) - piperazinyl) - quinoline - 3 - carboxylic acid, 1 - p - fluorophenyl - 6 - fluoro - 1,4-dihydro - 4 - oxo - 7 - (1 - piperazinyl) - quinoline - 3 - carboxylic acid, 1 - p - fluorophenyl - 6 - fluoro - 1,4 - dihydro - 4 - oxo - 7 - (1(4 - methyl) - piperazinyl) - quinoline - 3 - carboxylic acid, 1 - p - bromophenyl - 6 - fluoro - 1,4 - dihydro - 4 oxo - 7 - (1 - piperazinyl) - quinoline - 3 - carboxylic acid, 1 - p - hydroxyphenyl - 6 - fluoro - 1,4 - dihydro - 4 - oxo - 7 - (1 - piperazinyl) - quinoline - 3 - carboxylic acid, 1 - (2,4 - difluorophenyl) - 6 - fluoro - 1,4 - dihydro - 4 - oxo - 7 - (1 - piperazinyl) - quinoline - 3 - carboxylic acid, 1 - p - fluorophenyl - 6 - fluoro - 1,4 - dihydro - 4 - oxo - 7 - (1 - (4 - acetyl) - piperazinyl) - quinoline - 3 - carboxylic acid, 1 - p - fluorophenyl - 6 - fluoro - 1,4 - dihydro - 4 - oxo - 7 - (3 - amino - 1 - pyrrolidinyl) - quinoline - 3 - carboxylic acid, 1 - p - fluorophenyl - 6 - fluoro - 1,4 - dihydro - 4 - oxo - 7 - (3-methylamino - 1 - pyrrolidinyl) - quinoline - 3 - carboxylic acid, 1 - p - fluorophenyl - 6 - fluoro - 1,4 - dihydro - 4 - oxo - 7 - (3 - dimethylamino - 1 - pyrrolidinyl) - quinoline - 3 - carboxylic acid and 1 - (2,4 - difluorophenyl) - 6 - fluoro - 1,4 - dihydro - 4 - oxo - 7 - (3 - dimethylamino - 1 - pyrrolidinyl) - quinoline - 3 - carboxylic acid.

As used herein, the term "pharmaceutically acceptable salts" means the nontoxic acid addition or alkaline earth metal salts of the compounds of Formula I. These salts can be prepared *in situ* during the final isolation and purification of the compounds of Formula I, or by separately reacting the base or acid functions with a suitable organic or inorganic acid or base, respectively. Representative acid addition salts include the hydrochloride, hydrobromide, sulfate, bisulfate, acetate, oxalate, valerate, oleate, palmitate, stearate, laurate, borate, benzoate, lactate, phosphate, tosylate, citrate, maleate, fumarate, succinate, tartrate, napsylate, glucoheptonate, lactobionate, lauryl sulfate salts and the like. Representative alkali metal or alkaline earth metal salts include the sodium, calcium, potassium, and magnesium salts, and the like.

It has been found that the preferred compounds of the invention possess antibacterial activity against a wide spectrum of gram positive and gram negative bacteria. The compounds of the invention are therefore useful in the antibiotic treatment of susceptible bacterial infections in both humans and animals. In addition, the compounds may be used in scrub solutions, for surface inhibition of bacterial growth, e.g., on counter surfaces, and the like. Susceptible organisms generally include those gram positive and gram negative, aerobic and anazerobic organisms whose growth can be inhibited by the compounds of the inventions, such as *Staphyloccus, Lactobacillus, Streptococcus, Sarcina, Escherichia, Enterobacter, Klebsiella, Pseudomonas, Acinetobacter, Proteus, Citrobacter, Nisseria, Baccullus, Bacteroides, Peptococcus, Clostridium, Salmonella, Shigella, Serratia, Haemophilus, Brucella,* and other organisms. In addition to exhibiting highly effective antibacterial activity, the compounds of the invention exhibit

increased and improved solubility characteristics as compared with prior quinoline-3-carboxylic acid compounds in the art.

The compounds of Formula I may also be formulated into compositions together with pharmaceutically acceptable carriers for parenteral injection, for oral administration in solid or liquid form, for rectal administration and the like.

Compositions according to the invention for parenteral injection may comprise pharmaceutically acceptable sterile aqueous or nonaqueous solutions, suspensions or emulsions. Examples of suitable nonaqueous carriers, diluents, solvents or vehicles include propylene glycol, polyethylene glycol, vegetable oils, such as olive oil, and injectable organic esters such as ethyl oleate. Such compositions may also contain adjuvants such as preserving, wetting, emulsifying, and dispersing agents. They may be sterilized, for example, by filtration through a bacteria-retaining filter, or by incorporating sterilizing agents in the form of sterile solid compositions which can be dissolved in sterile water, or some other sterile injectable medium immediately before use.

Solid dosage forms for oral administation include capsules, tablets, pills, powders and granules. In such solid dosage forms, the active compound is admixed with at least one inert diluent such as sucrose, lactose or starch. Such dosage forms can also comprise, as in normal practice, additional substances other than inert diluents, e.g., lubricating agents such as magnesium stearate. In the case of capsules, tablets and pills, the dosage forms may also comprise buffering agents. Tablets and pills can additionally be prepared with enteric coatings.

Liquid dosage forms for oral administration include pharmaceutically acceptable emulsions, solutions, suspensions, syrups and elixirs containing inert diluents commonly used in the art, such as water. Besides such inert diluents, compositions can also include adjuvants, such as wetting agents, emulsifying and suspending agents, and sweetening, flavoring and perfuming agents.

Compositions for rectal administration are preferably suppositories which may contain, in addition to the active substance, excipients such as cocoa butter or a suppository wax.

Actual dosage levels of active ingredient in the compositions of the invention may be varied so as to obtain an amount of active ingredient effective to achieve antibacterial activity in accordance with the desired method of administration. The selected dosage level therefore depends upon the nature of the active compound administered, the route of administration, the desired duration of treatment and other factors. Generally, daily dosage levels of the compounds of Formula I of about 0.1 to about 750, more preferably about 0.25 to about 500 and most preferably about 0.5 to about 300 mg. of active ingredient per kg. of body weight are effective when administered orally to a mammalian patient suffering from an infection caused by a susceptible organism. If desired, the daily dose may be divided into multiple doses for administration, e.g., two to four times per day.

The compounds of Formula I may be prepared in accordance with the following reaction scheme:

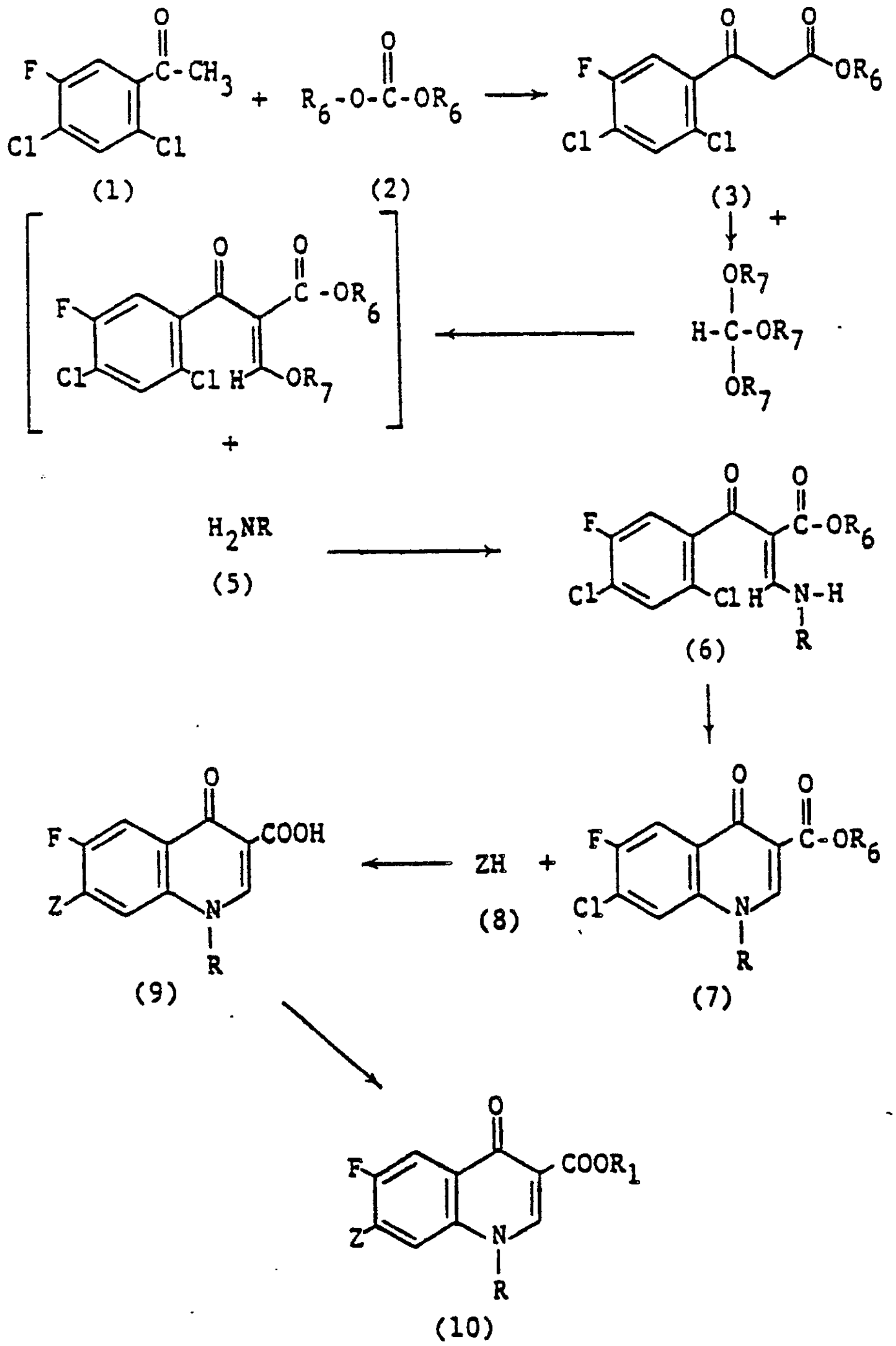

(1) (2) (3) (5) (6) (7) (8) (9) (10)

In accordance with the foregoing reaction scheme, 2,4-dichloro-5-fluoro-acetophenone (1) is reacted with a dialkoxycarbonate (2) in the presence of a strong base to obtain the corresponding B-ketoester (3). In the dialkoxycarbonate (2), $R_6$ may be an alkyl group of, for example, 1 to 10 carbon atoms, but is preferably lower alkyl, such as ethyl. Suitable bases include the metal hydrides, such as sodium hydride, potassium hydride and the like, as well as metal alkoxides in alcohol, such as sodium ethoxide in ethanol. The presently preferred base for this purpose is sodium hydroxide. Formation of the B-ketoester (3) is facilitated by reacting the acetophenone (1) with the dialkoxycarbonate (2) at elevated temperatures, such as from about 20°C to about 120°C, and preferably from about 30°C to about 90°C until completion of the reaction. The B-ketoester may then be separated from the reaction mixture in conventional manner.

The B-ketoester (3) is then treated with a trialkylorthoformate (4) in the presence of an acid anhydride, preferably acetic anhydride, followed by reaction with substituted or unsubstituted aniline or other aromatic heterocyclic amine (5) to obtain the enaminoketoester (6). In the trialkylorthoformate (4). $R_7$ may be an alkyl group of, for example, from 1 to 10 carbon atoms, but is preferably lower alkyl, such as ethyl. Reaction with the trialkylorthoformate is preferably conducted at elevated temperatures, such as from

about 50° to about 150°C, preferably from about 100°C to about 140°, to obtain an oily liquid as an unisolated intermediate (shown in brackets in the reaction scheme). Reaction of the latter with the substituted or unsubstituted aniline or aromatic heterocyclic amine (5) is preferably conducted in an appropriate aprotic or nonaprotic solvent, preferably methylene chloride or tetrahydrofuran, and may be conducted at room or suitable elevated temperature, as desired.

The enaminoketoester (6) is then cyclized, such as by treatment with a strong base as defined above, preferably sodium hydride, to obtain the 1-aryl-1,4-dihydro-4-oxo-quinoline-3-carboxylic acid ester (7). Cyclization is conducted in the presence of an aprotic solvents such as dimethoxyethane, dimethylformamide, tetrahydrofuran or chlorobenzene, and is preferably conducted at temperatures of about 20°C to about 145°C, more preferably at the reflux temperature of the solvent employed.

The ester (7) is subjected to hydrolysis, such as by treatment with sodium hydroxide, to form the free acid, followed by displacement of the 7-chloro radical with substituted or unsubstituted piperazine or other heterocyclic amines (8) as described above by techniques known in the art to obtain the desired 1-aryl-6-fluoro-7-substituted amino-1,4-dihydroxy-4-oxo-quinoline-3-carboxylic acid (9).

The 1-aryl-6-fluoro-7-substituted amino-1,4-dihydro-4-oxo-quinoline-3-carboxylic acid (9) can then be converted into the corresponding ester (10), if desired, by conventional esterification procedures, such as by treating the free acid (9) with the appropriate alcohol in the presence of an acid catalyst, by converting the free acid (9) into the corresponding acid chloride followed by displacement of the chloro radical with the appropriate alcohol, or by treating the sodium salt of the acid (9) with a suitable reactive halide, such as chloro-methyl-pivalate in dimethoxyethane to obtain, for example, the pivaloyloxymethyl ester (1) wherein $R_2$ is $—CH_2OCOC(CH_3)_3$.

The foregoing may be better understood from the following examples, which are presented for purposes of illustration and are not intended to limit the scope of the inventive concepts. As used in the following examples, the references to compounds, such as (1), (2), (3), etc., and to substituents, such as R, $R_1$, $R_2$, etc., refer to the correspondings and substituents in the foregoing scheme.

Example 1

1-phenyl-6-fluoro-1,4-dihydro-4-oxo-7 (1-piperazinyl) quinoline-3-carboxylic acid

a) To a cold solution of 20.5 g. 2,4-dichloro-5-fluoroacetophenone in 300 ml. diethylcarbonate is slowly added 8.2 g. 60% sodium hydride-in-oil suspension. The mixture is heated at 80°C for 1-1/2 hours, then poured into 700 ml. ice cold water solution containing 25 ml. acetic acid. The mixture is extracted with three 400 ml. portions of ether. The organic phase is dried over $MgSO_4$, evaporated and the obtained liquid is distilled at 111°C at 0.7 mm. of Hg. pressure to give 22.2 g. of (3) wherein $R_6=C_2H_5$.

b) A solution of 15.18 g. of B-ketoester (3) ($R_6=C_2H_5$) in 14 ml. of triethylorthoformate and 35 ml. of acetic anhydride is heated at 135°C for 1-1/2 hours with the removal of the ethyl acetate formed during the reaction. The solution is evaporated under reduced pressure to a mobile oil, The oil is then dissolved in 150 ml. of methylene chloride and 7.5 ml. of aniline is added into the solution. After 1 hour, the solution is evaporated to dryness and crystallized from 200 ml. of hexane and 5 ml. of ether yielding (6), wherein $R_6=C_2H_5$and R=phenyl in 89% yield, m.p. 96-97°C.

c) To a cold solution of 13.9 g. of the preceding product (6), $R_6=C_2H_5$, R=phenyl in 140 ml. dimethoxymethane (DME) is slowly added 1.49 g. of a 60% sodium hydride-in-oil suspension. The mixture is refluxed for 4-1/2 hours and is cooled and diluted with water to a volume of 1.5 liters. The mixture is then filtered and the solid is washed with a 1:1 hexane/ether solution to obtain 10.4 g. of (7) wherein $R_6=C_2H_5$and R=phenyl in 81% yield.

d) To a suspension of 5.4 g. of (7) ($R_6=C_2H_5$, R=phenyl) in 30 ml. THF is added a sodium hydroxide solution (0.73 g. in 20 ml. $H_2O$). The mixture is heated at 80°C for 1 hour resulting in a clear solution which is evaporated under reduced pressure to dryness. The solid is dissolved in 200 ml. $H_2O$, and 2 ml. acetic acid is added. The resulting precipitate is filtered and washed with cold water, crystallized from dimethylformamide (DMF) to produce 4.6 g. of 7-chloro-1-phenyl-6-fluoro-1,4-dihydro-4-oxo-quinoline-3-carboxylic acid (7) ($R_6$=hydrogen, R=phenyl), m.p. 271—273°C.

e) To a solution of 1.25 g. of 7-chloro-1-phenyl-6-fluoro-1,4-dihydro-4-oxo-quinoline-3-carboxylic acid in 15 ml. of 1-methyl-2-pyrrolidine at 115°C is added 2 ml. piperazine. After stiring at 100°C for 20 hours, the solvent is removed by reduced pressure to dryness. Ethanol is added to the residue and the resulting mixture is filtered and washed with ether and then washed with very small amounts of cold water. The resulting dried solid is suspended in 30 ml. $H_2O$ and 2.35 ml. 1N HCl is added to and warmed to dissolve. Removal of the solvent under reduced presssure gives 835 mg. hydrochloride salt of 1-phenyl-6-fluoro-1,4-dihydro-4-oxo-7-(1-piperazinyl)-quinoline-3-carboxylic acid (9) (R=phenyl,

Z= 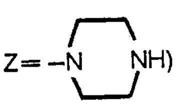 ) .

To the hydrochloride salt is added one molar equivalent of an aqueous solution of sodium hydroxide, and the resulting precipitate is filtered to obtain 1-phenyl-6-fluoro-1,4-dihydro-4-oxo-7-(1-piperazinyl)-quinoline-3-carboxylic acid.

f) Alternatively, the title compound is prepared as follows: To a suspension of 5.87 g. of compound (7) ($R_6$=H,R=phenyl; product of 1(d)) in 40 ml. 1-methyl-2-pyrrolidinone at 120°C under nitrogen atmosphere is added 9.5 ml. of N-carboethoxy-p-piperazine. After 20 hours, the solvent is removed under reduced pressure, and the residue is suspended in 150 ml. ethanol and refluxed for 1/2 hour. The reaction mixture is then cooled and filtered. The resulting solid is washed with cold ethanol and water to obtain 6.15 g. of compound (9) (R=phenyl,

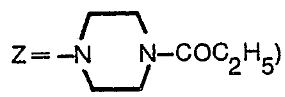

in 87 % yield.

To a suspension of 5.5 g. of the preceding compound (9) (R=phenyl,

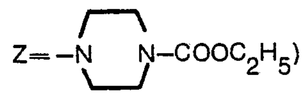

in 25 ml. of ethanol at 80°C is added 50 ml. of 10% NaOH solution. The solution is heated at 80°C for 6 hours. The solvent is removed and the solid is dissolved in 100 ml. water. The pH of the solution is adjusted to pH 7 by the addition of 10% acetic acid. The precipitate is filtered and washed with cold water yielding 4.26 of 1-phenyl-6-fluoro-1,4-dihydro-4-oxo-7-(1-piperazinyl)quinoline-3-carboxylic acid (9) (R=phenyl,

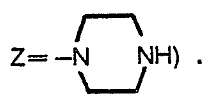

Example 2

1-phenyl-6-fluoro-1,4-dihydro-4-oxo-7-(1-(4-methyl)-piperazinyl)quinoline-3-carboxylic acid

The procedure of Example 1 can be repeated, replacing piperazine in Example 1(e) with N-methylpiperazine to obtain 1-phenyl-6-fluoro-1,4-dihydro-4-oxo-7-(1-(4-methyl)-piperazinyl)-quinoline-3-carboxylic acid (9) (R=phenyl

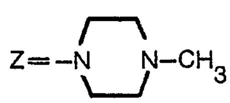

and its hydrochloride salt.

Example 3

1-phenyl-6-fluoro-1,4-dihydro-4-oxo-7-(1-(4-ethyl)-piperazinyl)quinoline-3-carboxylic acid

The procedure of Example 1 can be repeated, replacing piperazine in Example 1(e) with N-ethylpiperazine to obtain the 1-phenyl-6-fluoro-1,4-dihydro-4-oxo-7-(1-(4-ethyl)-piperazinyl)-quinoline-3-carboxylic acid (9) (R=phenyl,

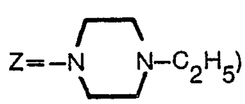

and its hydrochloride salt.

Example 4

1-p-fluorophenyl-6-fluoro-1,4-dihydro-4-oxo-(1-piperazinyl)quinoline-3-carboxylic acid

a) In the described fashion as Example 1(b), replacing aniline with p-fluoroaniline, one can obtain the enaminoketoester (6) $R_6$—$C_2H_5$, R=p-fluorophenyl) in 78% yield, m.p. 108°C.

b) By following the Example 1(c) and 1(d), the preceding compound (6) $R_6$=$C_2H_5$, R=p-fluorophenyl) can yield 7-chloro-1-p-fluorophenyl-6-fluoro-1,4-dihydro-4-oxo-quinoline-3-carboxylic acid (7) ($R_6$=H, R=p-fluorophenyl).

c) In the described fashion as Example 1(e), the above acid (7) ($R_6$=H, R=p-fluorophenyl) can give the desired 1-p-fluoro-phenyl-6-fluoro-1,4-dihydro-4-oxo-7-(1-piperazinyl)quinoline-3-carboxylic acid (9)

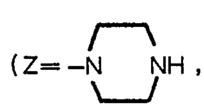

R=p-fluorophenyl) and its hydrochloride salt.

Example 5

1-p-fluorophenyl-6-fluoro-1,4-dihydro-4-oxo-7-(1-(4-methyl)-piperazinyl)quinoline-3-carboxylic acid

In the described fashion as Example 1(e), replacing piperazine with N-methylpiperazine, the acid (7) ($R_6$=H, R=p-fluorophenyl in Example 4(b) can yield the described 1-p-fluorophenyl-6-fluoro-1,4-dihydro-4-oxo-7-(1-(4-methyl)-piperazinyl)quinoline-3-carboxylic acid (9)

( Z= 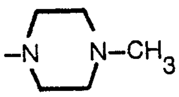 ,

R=p-fluorophenyl) and its hydrochloride salt.

Example 6

1-p-chlorophenyl-6-fluoro-1,4-dihydro-4-oxo-(1-piperazinyl)quinoline-3-carboxylic acid

a) In the described fashion as Example 1(b), replacing aniline with p-chloroaniline, one can obtain the enaminoketoester (6) $R_6=C_2H_5$, R=p-chlorophenyl) in 64% yield.

b) By following Example 1(c) and 1(d), the preceding compound (6) $R_6=C_2H_5$, R=p-chlorophenyl) can yield 7-chloro-1-p-chlorophenyl-6-fluoro-1,4-dihydro-4-oxo-quinoline-3-carboxylic acid (7) ($R_6$=H, R-p-chlorophenyl).

c) In the described fashion as Example 1(e), the above acid (7) ($R_6$=H, R=p-chlorophenyl) can give the desired 1-p-chlorophenyl-6-fluoro-1,4-dihydro-4-oxo-7-(piperazinyl)quinoline-3-carboxylic acid (9)

( Z= 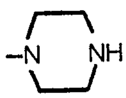 ,

R=p-chlorophenyl) and its hydrochloride salt.

Example 7

1-p-chlorophenyl-6-fluoro-1,4-dihydro-4-oxo-7-(1-(4-methyl)-piperazinyl)quinoline-3-carboxylic acid

In the described fashion as Example 1(e), replacing piperazine with N-methylpiperazine, the acid (7) ($R_6$=H, R=p-chlorophenyl in Example 6(b) can yield the desired 1-p-chlorophenyl-6-fluoro-1,4-dihydro-4-oxo-7-(1-(4-methyl)-piperazinyl)quinoline-3-carboxylic acid (9)

(Z= 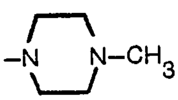 ,

R=p-chlorophenyl) and its hydrochloride salt.

Example 8

1-p-methoxyphenyl-6-fluoro-1,4-dihydro-4-oxo-(1-piperazinyl)quinoline-3-carboxylic acid

a) In the described fashion as Example 1(b), replacing aniline with p-methoxyaniline, one can obtain the enaminoketoester (6) $R_6=C_2H_5$, R=p-$OCH_3$-phenyl) in 77% yield (m.p. 105°C).

b) By following the Example 1(c) and 1(d), the preceding compound (6) $R_6=C_2H_5$, R=p-methoxyphenyl) can yield 7-chloro-1-p-methoxyphenyl-6-fluoro-1,4-dihydro-4-oxo-quinoline-3-carboxylic acid (7) ($R_6$=H, R=p-methoxyphenyl).

c) In the described fashion as Example 1(e), the above acid (7) ($R_6$=H, R=p-methoxyphenyl) can give the desired 1-p-methoxyphenyl-6-fluoro-1,4-dihydro-4-oxo-7-(1-piperazinyl)-quinoline-3-carboxylic acid (9)

(Z= 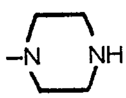 ,

R=p-methoxyphenyl) and its hydrochloride salt.

Example 9

1-p-methoxyphenyl-6-fluoro-1,4-dihydro-4-oxo-7-(1-(4-methyl)-piperazinyl)quinoline-3-carboxylic acid

In the described fashion as Example 1(e), replacing piperazine with N-methylpiperazine, the acid (7) ($R_6$=H, R=p-methoxyphenyl in Example 8(b) can yield the described 1-p-methoxyphenyl-6-fluoro-1,4-dihydro-4-oxo-7-(1-(methyl)-piperazinyl)quinoline-3-carboxylic acid (9)

(Z= 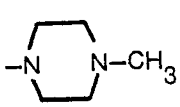 ,

R=p-chlorophenyl) and its hydrochloride salt.

Example 10

1-p-methylphenyl-6-fluoro-1,4-dihydro-4-oxo-7-(piperazinyl)quinoline-3-carboxylic acid

a) In the described fashion as Example 1(b), replacing aniline with p-methylaniline, one can obtain the enaminoketoester (6) $R_6=C_2H_5$, R=p-methylphenyl) in 80% yield (m.p. 115.5°C).

b) By following the Example 1(c) and 1(d), the preceding compound (6) $R_6=C_2H_5$, R=p-methylphenyl) can yield 7-chloro-1-p-methylphenyl-6-fluoro-1,4-dihydro-4-oxo-quinoline-3-carboxylic acid (7) ($R_6$=H, R=p-methylphenyl).

c) In the described fashion as Example 1(e), the above acid (7) ($R_6$=H, R=p-methylphenyl) can give the desired 1-p-methylphenyl-6-fluoro-1,4-dihydro-4-oxo-7-(1-piperazinyl)-quinoline-3-carboxylic acid (9)

(Z= 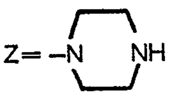 ,

R=p-methylphenyl) and its hydrochloride salt.

Example 11

1-p-methylphenyl-6-fluoro-1,4-dihydro-4-oxo-7-(1-(4-methyl)-piperazinyl)quinoline-3-carboxylic acid

In the described fashion as Example 1(e), replacing piperazine with N-methylpiperazine, the acid (7) ($R_6$=H, R=p-methylphenyl in Example 10(b) can yield the desired 1-p-methylphenyl-6-fluoro-1,4-dihydro-4-oxo-7-(1-(4-methyl)-piperazinyl)quinoline-3-carboxylic acid (9)

(Z= 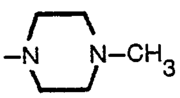 ,

R=p-methylphenyl) and its hydrochloride salt.

Example 12

1-p-hydroxyphenyl-6-fluoro-1,4-dihydro-4-oxo-(1-piperazinyl)quinoline-3-carboxylic acid

a) In the described fashion as Example 1(b), replacing aniline with p-hydroxyaniline, one can obtain the enaminoketoester (6) $R_6=C_2H_5$, R=p-hydroxyphenyl) in 84% yield.

b) By following the Example 1(c) and 1(d), the preceding compound (6) $R_6=C_2H_5$, R=p-hydroxyphenyl) can yield 7-chloro-1-p-hydroxyphenyl-6-fluoro-1,4-dihydro-4-oxo-quinoline-3-carboxylic acid (7) ($R_6$=H, R=p-hydroxyphenyl) in good yield.

c) In the described fashion as Example 1(e), the above acid (7) ($R_6$=H, R=p-hydroxyphenyl) can give the desired 1-p-hydroxyphenyl-6-fluoro-1,4-dihydro-4-oxo-7-(1-piperazinyl)-quinoline-3-carboxylic acid (9)

(Z= 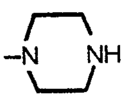 ,

R=p-hydroxyphenyl) and its hydrochloride salt.

Example 13

1-p-hydroxyphenyl-6-fluoro-1,4-dihydro-4-oxo-7-(1-(4-methyl)-piperazinyl)quinoline-3-carboxylic acid

In the described fashion as Example 1(e), replacing piperazine with N-methylpiperazine, the acid (7) ($R_6$=H, R=p-hydroxyphenyl) in Example 12(b) can yield the desired 1-p-hydroxyphenyl-6-fluoro-1,4-dihydro-4-oxo-7-(1-(4-methyl)-piperazinyl)quinoline-3-carboxylic acid (9)

(Z= 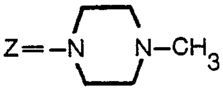 ,

R=p-hydroxyphenyl) and its hydrochloride salt.

Example 14

1-o-fluorophenyl-6-fluoro-1,4-dihydro-4-oxo-(1-piperazinyl)quinoline-3-carboxylic acid

a) In the described fashion as Example 1(b), replacing aniline with o-fluoroaniline, one can obtain the enaminoketoester (7) ($R_6=C_2H_5$, R=o-fluorophenyl) in 78.8% yield (m.p. 90—92°C).

b) By following the Example 1(c) and 1(d), the preceding compound (6) ($R_6=C_2H_5$, R=o-fluorophenyl) can yield 7-chloro-1-o-fluorophenyl-6-fluoro-1,4-dihydro-4-oxo-quinoline-3-carboxylic acid (7) ($R_6$=H, R=o-fluorophenyl).

c) In the described fashion as Example 1(e), the above acid (7) ($R_6$=H, R=o-fluorophenyl) can give the desired 1-o-fluorophenyl-6-fluoro-1,4-dihydro-4-oxo-7-(1-piperazinyl)-quinoline-3-carboxylic acid (9)

(Z= 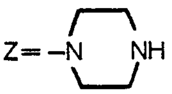 ,

R=o-fluorophenyl) and its hydrochloride salt.

Example 15

1-o-fluorophenyl-6-fluoro-1,4-dihydro-4-oxo-7-(1-(4-methyl)-piperazinyl)quinoline-3-carboxylic acid

In the described fashion as Example 1(e), replacing piperazine with N-methylpiperazine, the acid (7) ($R_6$=H, R=o-fluorophenyl) in Example 14(b) can yield the desired 1-o-fluorophenyl-6-fluoro-1,4-dihydro-4-oxo-7-(1-(4-methyl)-piperazinyl)quinoline-3-carboxylic acid (9)

(Z= 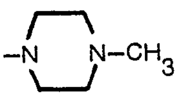 ,

R=o-fluorophenyl) and its hydrochloride salt.

Example 16

1-m-fluorophenyl-6-fluoro-1,4-dihydro-4-oxo-(1-piperazinyl)quinoline-3-carboxylic acid

a) In the described fashion as Example 1(b), replacing aniline with m-fluoroaniline, one can obtain the enaminoketoester (6) ($R_6$=$C_2H_5$, R=m-fluorophenyl) in 68.5% yield (m.p. 103—104°C).

b) By following the Example 1(c) and 1(d), the preceding compound (6) ($R_6$=$C_2H_5$, R=m-fluorophenyl) can yield 7-chloro-1-m-fluorophenyl-6-fluoro-1,4-dihydro-4-oxo-quinoline-3-carboxylic acid (7) ($R_6$=H, R=m-fluorophenyl).

c) In the described fashion as Example 1(e), the above acid (7) ($R_6$=H, R=m-fluorophenyl) can give the desired 1-m-fluorophenyl-6-fluoro-1,4-dihydro-4-oxo-7-(1-piperazinyl)-quinoline-3-carboxylic acid (9)

(Z= 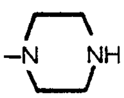 ,

R=m-fluorophenyl) and its hydrochloride salt.

Example 17

In a similar fashion as Example 1, the use of various substituted anilines and other amino aromatic compounds in place of aniline and the use of appropriate N-substituted piperazine, the following additional compounds and their hydrochloride salts can be made as summarized in Table I.

Table I

| | Aniline Replacement | Piperazine Substituent | Compound (9) Obtained Z | R |
|---|---|---|---|---|
| 1. | p-trifluoro-methylaniline | H | piperazinyl | p-trifluoro-methylphenyl |
| 2. | 2,4-difluoro-aniline | H | piperazinyl | 2,4-difluorophenyl |
| 3. | 2,4-difluoro-aniline | $CH_3$ | N-methyl-piperazinyl | 2,4-difluoro-phenyl |
| 4. | 2-chloro-4-fluoroaniline | H | piperazinyl | 2-chloro-4-fluorophenyl |
| 5. | 2-fluoro-4-hydroxyaniline | H | piperazinyl | 2-fluoro-4-hydroxyphenyl |
| 6. | 3,4-methylene-dioxyaniline | H | piperazinyl | p-fluorophenyl |
| 7. | 2,4-dihydroxy-aniline | H | piperazinyl | 2,4-hydroxyphenyl |
| 8. | 3-fluoro-4-hydroxyaniline | H | piperazinyl | 3-fluoro-4-hydroxyphenyl |
| 9. | 3-chloro-4-hydroxyaniline | H | piperazinyl | 3-chloro-4-hydroxyphenyl |
| 10. | 3,4-methylene-dioxyaniline | H | piperazinyl | 3,4-methylene-dioxyphenyl |
| 11. | 3,4-methylene-dioxyaniline | H | N-methyl-piperazinyl | 3,4-methylene-dioxyphenyl |

Example 18 (Reference example only)

1-p-fluorophenyl-6-fluoro-1,4-dihydro-4-oxo-7-thiomorpholino-quinoline-3-carboxylic acid

To a solution of 3.36 g. of 7-chloro-1-p-fluoro-phenyl-6-fluoro-4-oxo-quinoline-3-carboxylic acid (the product of Example 4 (b)) in 35 ml. of 1-methyl-2-pyrrolidinone at 115°C is added 4.13 g. of thiomorpholine. After stirring at 115°C for 20 hours, the solvent is removed by reduced pressure to dryness. Methanol is added to the residue and the resulting mixture is filtered and washed with ethylacetate and then washed with water. The solid is dried, yielding 2.51 g. 1-p-fluorophenyl-6-fluoro-1,4-dihydro-4-oxo-7-thiomorpholino-quinoline-3-carboxylic acid (9)

(Z=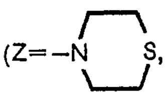,

R=p-fluorophenyl) in 62% yield.

Example 19 (Reference example only)

1-phenyl-6-fluoro-1,4-dihydro-4-oxo-7-morpholino-quinoline-3-carboxylic acid

To a solution of 2.5 g. of 7-chloro-1-phenyl-6-fluoro-1,4-dihydro-4-oxo-quinoline-3-carboxylic acid in 30 ml. of 1-methyl-2-pyrrolidinone at 115°C is added in 4 ml. of morpholine. After stirring at 115°C for 20 hours, the solvent is removed by reduced pressure to dryness. Ethanol is added to the residue and the resulting mixture is filtered and washed with ethanol and then water. The solid is dried, yielding 1.98 g. 1-phenyl-6-fluoro-1,4-dihydro-4-oxo-7-morpholino-quinoline-3-carboxylic acid (9)

(Z= 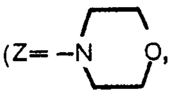,

R=phenyl).

Example 20

1-phenyl-6-fluoro-1,4-dihydro-4-oxo-7-(3-amino-1-pyrrolidinyl)quinoline-3-carboxylic acid

a) To a solution of 1.5 g. of 7-chloro-1-phenyl-6-fluoro-1,4-dihydro-4-oxo-quinoline-3-carboxylic acid in 20 ml. of 1-methyl-2-pyrrolidone at 115°C is added 3 g. of 3-acetamido-pyrrolidone. After stirring at 100°C for 20 hours, the solvent is removed by reduced pressure to dryness. Ethanol is added to the residue and washed with ether and then diluted cold hydrochloric acid and then water. The solid is dried, yielding 1.60 g. 1-phenyl-6-fluoro-1,4-dihydro-4-oxo-7-(3-acetamido-1-pyrrolidinyl)-quinoline-3-carboxylic acid (9)

(Z= 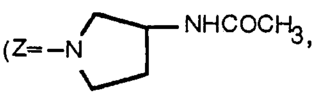,

R=phenyl).

b) The above product is then hydrolysed with hydrochloric acid at 80°C to give 1-phenyl-6-fluoro-1,4-dihydro-4-oxo-7-(3-amino-1-pyrrolidinyl)quinoline-3-carboxylic acid (9)

(Z= 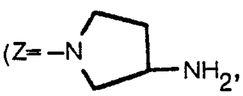,

R=phenyl) hydrochloride salt.

Example 21

1-p-fluorophenyl-6-fluoro-1,4-dihydro-4-oxo-7-(3-amino-1-pyrrolidinyl)quinoline-3-carboxylic acid

In the described fashion as Example 20, replacing the 7-chloro-1-phenyl-6-fluoro-1,4-dihydro-4-oxo-quinoline-3-carboxylic acid with 7-chloro-1-p-fluorophenyl-6-fluoro-1,4-dihydro-4-oxo-quinoline-3-carboxylic acid, one can obtain the 1-p-fluorophenyl-6-fluoro-1,4-dihydro-4-oxo-7-(3-amino-1-pyrrolidinyl)quinoline-3-carboxylic acid hydrochloride salt (9)

(Z= 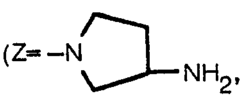,

R=p-fluorophenyl) in good yield.

Example 22

1-p-hydroxyphenyl-6-fluoro-1,4-dihydro-4-oxo-7-(3-amino-1-pyrrolidinyl)quinoline-3-carboxylic acid

In the described fashion as Example 20, replacing the 7-chloro-1-phenyl-6-fluoro-1,4-dihydro-4-oxo-quinoline-3-carboxylic acid with 7-chloro-1-p-hydroxyphenyl-6-fluoro-1,4-dihydro-4-oxo-quinoline-3-carboxylic acid, one can obtain the 1-p-hydroxyphenyl-6-fluoro-1,4-dihydro-4-oxo-7-(3-amino-1-pyrrolidinyl)quinoline-3-carboxylic acid hydrochloride salt (9)

(Z= 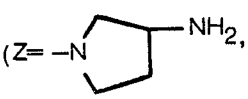,

R=p-fluorophenyl) in good yield.

Example 23

1-o,p-difluorophenyl-6-fluoro-1,4-dihydro-4-oxo-7-(3-amino-1-pyrrolidinyl)quinoline-3-carboxylic acid

In the described fashion as Example 20, replacing the 7-chloro-1-phenyl-6-fluoro-1,4-dihydro-4-oxo-quinoline-3-carboxylic acid with 7-chloro-1-o, p-dihydrophenyl-6-fluoro-1,4-dihydro-4-oxo-quinoline-3-carboxylic acid, one can obtain the 1-o, p-difluorophenyl-6-fluoro-1,4-dihydro-4-oxo-7-(3-amino-1-pyrrolidinyl)quinoline-3-carboxylic acid and its hydrochloride salt (9)

(Z= 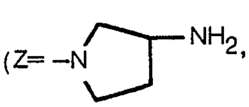,

R=2,4-difluorophenyl).

Example 24

1-p-fluorophenyl-6-fluoro-1,4-dihydro-4-oxo-7-(3-amino-1-pyrrolidinyl)quinoline-3-carboxylic acid

The procedure of Reference Example 18 can be repeated, replacing thiomorpholine with 3-dimethylamino pyrrolidine to obtain 1-p-fluorophenyl-6-fluoro-1,4-dihydro-4-oxo-7-(3-dimethylamino-1-pyrrolidinyl)quinoline-3-carboxylic acid and its hydrochloride salt (9)

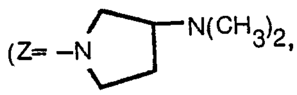

R=4-fluorophenyl).

Example 25

1-p-fluorophenyl-6-fluoro-1,4-dihydro-4-oxo-7-(3-methylamino-1-pyrrolidinyl)quinoline-3-carboxylic acid

a) The procedure of Example 24 can be repeated, replacing 3-dimethylamino pyrrolidine with 3-N-methylacetamidopyrrolidine to obtain 1-p-fluorophenyl-6-fluoro-1,4-dihydro-4-oxo-7-(3-N-methyl-acetamidopyrrolidinyl)quinoline-3-carboxylic acid (9) Z=N—NHCOCH$_3$, R=4-fluorophenyl.

b) The above product is then hydrolysed with hydrochloric acid at 80°C to give 1-p-fluorophenyl-6-fluoro-1,4-dihydro-4-oxo-7-(3-methylaminopyrrolidinyl)quinoline-3-carboxylic acid.

Example 26

In similar fashion as Reference Example 19, the use of various 1-aryl-7-chloro-6-fluoro-1,4-dihydro-4-oxo-quinoline-3-carboxylic acids (7), wherein aryl represents R, in place of 7-chloro-1-phenyl-6-fluoro-1,4-dihydro-4-oxo-quinoline-3-carboxylic acid (7) (R=phenyl) and the use of appropriate substituted amines in place of morpholine, the following additional compounds can be made as summarized in Table III.

Table III

| | Morpholine Replacement | Acid (7) $R_6$=H R | Compound (9) Obtained Z | R |
|---|---|---|---|---|
| 1. | 3-hydroxy-pyrrolidine | phenyl | 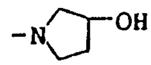 | phenyl |
| 2. | 3-hydroxy-pyrrolidine | p-fluorophenyl | 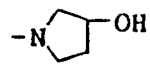 | p-fluorophenyl |
| 3. | 3-hydroxy-pyrrolidine | p-hydroxyphenyl | 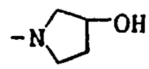 | p-hydroxyphenyl |
| 4. | 3-hydroxy-pyrrolidine | p-methylphenyl | 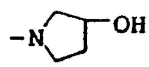 | p-methylphenyl |
| 5. | pyrrolidine | phenyl | 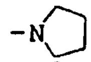 | phenyl |
| 6. | pyrrolidine | p-fluorophenyl | 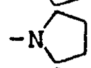 | p-fluorophenyl |
| 7. | pyrrolidine | p-hydroxyphenyl | 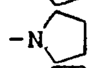 | p-hydroxyphenyl |
| 8. | azetidine | p-fluorophenyl | 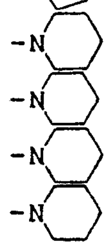 | p-fluorophenyl |
| 9. | piperidine | phenyl | | phenyl |
| 10. | piperidine | p-fluorophenyl | | p-fluorophenyl |
| 11. | piperidine | p-hydroxyphenyl | | p-hydroxyphenyl |
| 12. | pyrrolidine | 2,4-difluorophenyl | 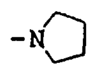 | 2,4-difluoro-phenyl |
| 13. | pyrrolidine | 2-hydroxy-4-fluorophenyl | 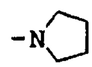 | 2-hydroxy-4-fluorophenyl |
| 14. | pyrrolidine | 4-hydroxy-2-fluorophenyl | 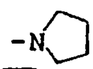 | 4-hydroxy-2-fluorophenyl |
| 15. | 4-acetylpiperazine | p-fluorophenyl | 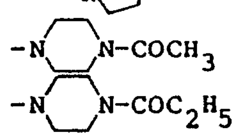 | p-fluorophenyl |
| 16. | 4-propionyl- | p-fluorophenyl | | p-fluorophenyl |

**Claims for the Contracting States: BE CH DE FR GB IT LI LU NL SE**

1. A compound having the formula:

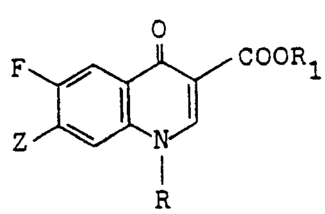

wherein $R_1$ is hydrogen or a carboxy protecting group; R is a phenyl group having the formula:

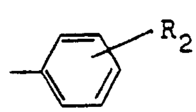

wherein $R_2$ is one or more groups selected from the group consisting of hydrogen, halogen, methylenedioxy, $C_1$ to $C_6$ alkyl, and a group having the formula:

$$-OR_3$$

wherein $R_3$ is hydrogen or $C_1$ to $C_6$ alkyl; and Z is a heterocyclic ring having the structure:

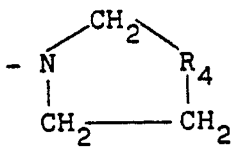

wherein $R_4$ is $CH_2$, $(CH_2)_2$ or $(CH_2)_nNH$— wherein n is 1 or 2, and substituted derivatives thereof, wherein said substituents are selected from one or more $C_1$ to $C_4$ alkyl and $-NR_5R_6$ wherein $R_5$ and $R_6$ are independently selected from hydrogen, $C_1$ to $C_4$ alkyl and alkanoyl; and pharmaceutically acceptable salts thereof.

2. A compound having the formula:

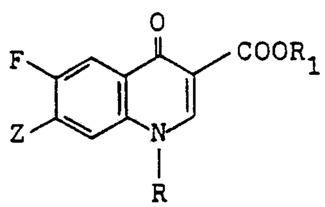

wherein Z is piperazinyl, $C_1$—$C_6$ alkyl-substituted piperazinyl, or amino-substituted pyrrolidinyl, R is phenyl or substituted phenyl wherein the substituent on the phenyl group is one or more of an $C_1$—$C_6$ alkyl, halo, methylenedioxy and hydroxy and $R_1$ is hydrogen or a carboxy protecting group.

3. A compound as defined in Claim 2 wherein R is phenyl, Z is piperazinyl and $R_1$ is hydrogen.

4. A compound as defined in Claim 2 wherein R is phenyl, Z is 4-methylpiperazinyl and $R_1$ is hydrogen.

5. A compound as defined in Claim 2 wherein R is p-fluorophenyl, Z is piperazinyl and $R_1$ is hydrogen.

6. A compound as defined in Claim 2 wherein R is p-fluorophenyl, Z is 4-methylpiperazinyl and $R_1$ is hydrogen.

7. A compound as defined in Claim 2 wherein Z is piperazinyl, R is 2,4-difluorophenyl and $R_1$ is hydrogen.

8. A compound as recited in Claim 2 wherein R is fluorophenyl, Z is methylpiperazinyl, and $R_1$ is hydrogen.

9. A compound as recited in Claim 2 wherein R is p-fluorophenyl, Z is 3 amino-1-pyrrolidinyl and $R_1$ is hydrogen.

10. A compound as defined in Claim 2 wherein Z is piperazinyl and R is difluorophenyl.

11. A compound as defined in Claim 2 wherein Z is methylpiperazinyl and R is difluorophenyl.

12. A compound as defined in Claim 2 wherein Z is aminopyrrolidinyl and R is 2,4-difluorophenyl.

13. A compound as defined in Claim 2 wherein Z is 3-methylpiperazinyl, R is 2,4-difluorophenyl and $R_1$ is hydrogen.

14. A composition having antibacterial activity in pharmaceutical dosage form containing a diluent and a compound as defined in Claim 1.

15. A composition having antibacterial activity in pharmaceutical dosage form containing a diluent and a compound defined in Claim 2.

16. A composition as defined in Claim 15 wherein Z is piperazinyl, R is p-fluorophenyl and $R_1$ is hydrogen.

17. A composition as defined in Claim 15 wherein Z is 4-methylpiperazinyl, R is p-fluorophenyl and $R_1$ is hydrogen.

18. A composition as defined in Claim 15 wherein Z is 3-methylpiperazinyl, R is 2,4-difluorophenyl and $R_1$ is hydrogen.

19. Use of a compound according to Claim 1 for manufacturing a drug for treating a bacterial infection in a patient.

20. Use of a compound according to Claim 2 for manufacturing a drug for treating a bacterial infection in a patient.

21. Use as defined in Claim 20 of a compound wherein Z is piperazinyl, R is p-fluorophenyl and $R_1$ is hydrogen.

22. Use as defined in Claim 20 of a compound wherein Z is 4-methylpiperazinyl, R is p-fluorophenyl and $R_1$ is hydrogen.

23. Use as defined in Claim 20 of a compound wherein Z is 3-methylpiperazinyl, R is 2,4-difluorophenyl and $R_1$ is hydrogen.

24. A method of producing 1,4-dihydro-4-oxoquinoline-3-carboxylic acids, comprising reacting the compound of the formula:

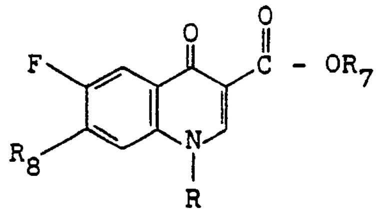

wherein $R_7$ is an alkyl group of 1 to 10 carbon atoms, $R_8$ is a leaving group and R is a phenyl group having the formula:

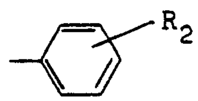

wherein $R_2$ is one or more groups selected from the group consisting of hydrogen, halogen, methylenedioxy, $C_1$ to $C_6$ alkyl and a group having the formula:

$$-OR_3$$

wherein $R_3$ is hydrogen or $C_1$ to $C_6$ alkyl; with a compound of the formula ZH wherein Z is a heterocyclic ring having the structure:

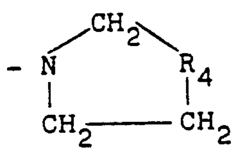

wherein $R_4$ is $CH_2$, $(CH_2)_2$ or $(CH_2)_nNH—$ wherein is 0, 1 or 2 and substituted derivatives thereof, wherein said substituents are selected from one or more of $C_1$ to $C_4$ alkyl and $—NR_5R_6$ wherein $R_5$ and $R_6$ are independently selected from hydrogen, $C_1$ to $C_4$ alkyl and alkanoyl.

**Claims for the Contracting State: AT**

1. A process for preparing a compound having the formula:

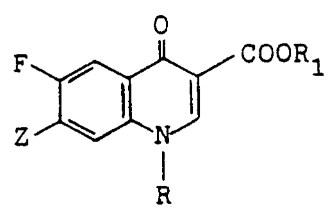

wherein $R_1$ is hydrogen or a carboxy protecting group; R is a phenyl group having the formula:

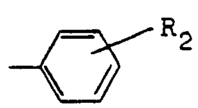

wherein $R_2$ is one or more groups selected from the group consisting of hydrogen, halogen, methylenedioxy, $C_1$ to $C_6$ alkyl, and a group having the formula:

$$-OR_3$$

wherein $R_3$ is hydrogen or $C_1$ to $C_6$ alkyl; and Z is a heterocyclic ring having the structure:

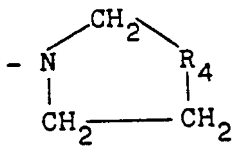

wherein $R_4$ is $CH_2$, $(CH_2)_2$ or $(CH_2)_nNH$—, wherein n is 1 or 2, and substituted derivatives thereof, wherein said substituents are selected from one or more $C_1$ to $C_4$ alkyl and —$NR_5R_6$ wherein $R_5$ and $R_6$ are indepenently selected from hydrogen, $C_1$ to $C_4$ alkyl and alkanoyl; and pharmaceutically acceptable salts thereof, comprising:

a) reacting 2,4-dichloro-5-fluoro-acetophenone (1) with dialkoxycarbonate (2) wherein $R_6$ is a $C_1$—$C_{10}$ alkyl, to obtain a corresponding β-ketoester (3):

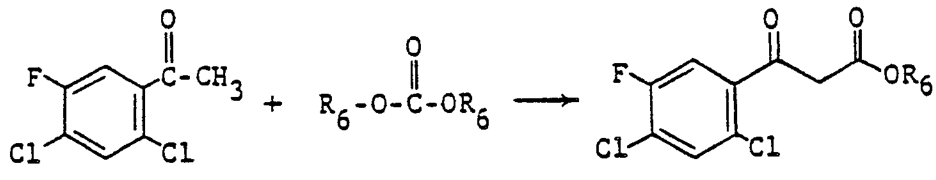

(1) (2) (3)

b) treating the β-ketoester (3) first with a trialkylorthoformate (4), wherein $R_7$ is a $C_1$—$C_{10}$ alkyl, and then with aniline $NH_2R$, wherein R is as defined above, to obtain an enaminoketoester (6):

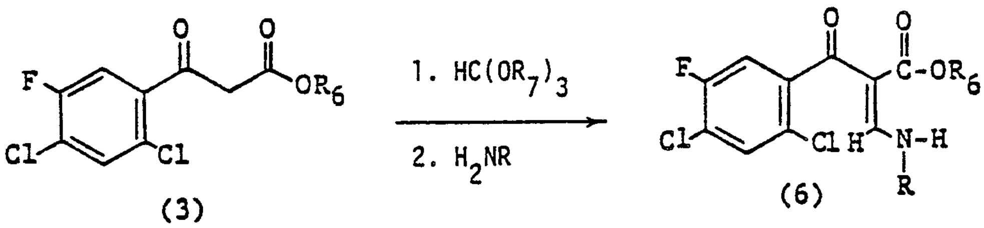


(3) (6)

c) cyclizing the enaminoketoester (6) to a corresponding quinoline-3-carboxylic acid ester (7)

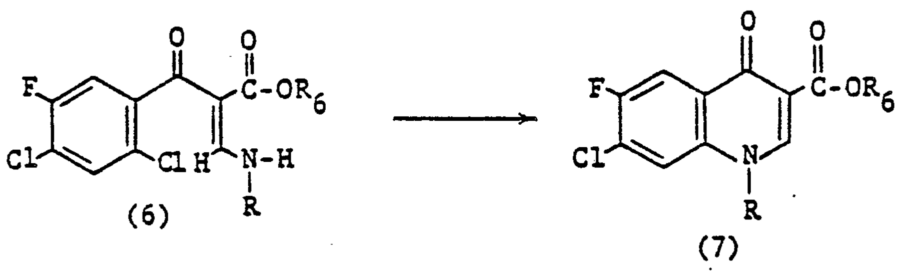

(6) (7)

d) hydrolising the ester (7) to its corresponding fred 3-carboxylic acid,

e) reacting said free acid from (d) with ZH (8) wherein Z is as defined above to obtain the compound I wherein $R_1$ is hydrogen, and possibly esterifying it to obtain a compound I wherein $R_1$ is different from a hydrogen.

2. A process according to claim 1, wherein Z is piperazinyl, $C_1$—$C_6$ alkyl-substituted piperazinyl, or amino-substituted pyrrolidinyl, R is phenyl or substituted phenyl wherein the substituent on the phenyl group is one or more of an $C_1$—$C_6$ alkyl, halo, methylenedioxy and hydroxy and $R_1$ is hydrogen or a carboxy protecting group.

3. A process as defined in Claim 2, wherein R is phenyl, Z is piperazinyl and $R_1$ is hydrogen.

4. A process as defined in Claim 2 wherein R is phenyl, Z is 4-methylpiperazinyl and $R_1$ is hydrogen.

5. A process as defined in Claim 2 wherein R is p-fluorophenyl, Z is piperazinyl and $R_1$ is hydrogen.

6. A process as defined in Claim 2, wherein R is p-fluorophenyl, Z is 4-methylpiperazinyl and $R_1$ is hydrogen.

7. A process as defined in Claim 2 wherein Z is piperazinyl, R is 2,4-difluorophenyl and $R_1$ is hydrogen.

8. A process as recited in claim 2 wherein R is fluorophenyl, Z is methylpiperazinyl, and $R_1$ is hydrogen.

9. A process as recited in claim 2 wherein R is p-fluorophenyl, Z is 3 amino-1-pyrrolidinyl and $R_1$ is hydrogen.

10. A process as defined in Claim 2, wherein Z is piperazinyl and R is difluorophenyl.

11. A process as defined in Claim 2 wherein Z is methylpiperazinyl and R is difluorophenyl.

12. A process as defined in Claim 2, wherein Z is aminopyrrolidinyl and R is 2,4-difluorophenyl.

13. A process as defined in Claim 2 wherein Z is 3-methylpiperazinyl, R is 2,4-difluorophenyl and $R_1$ is hydrogen.

14. A process for preparing a composition having antibacterial activity comprising mixing a diluent with a compound obtained by a process according to claim 1.

15. A process for preparing a composition having antibacterial activity comprising mixing a diluent with a compound prepared with the process according to claim 2.

16. A process as defined in claim 15 wherein in said compound Z is piperazinyl, R is p-fluorophenyl and $R_1$ is hydrogen.

17. A process as defined in claim 15 wherein in said compound Z is 4-methylpiperazinyl, R is p-fluorophenyl and $R_1$ is hydrogen.

18. A process as defined in Claim 15 wherein in said compound Z is 3-methylpiperazinyl, R is 2,4-difluorophenyl and $R_1$ is hydrogen.

**Patentansprüche für die Vertragsstaaten: BE CH DE FR GB IT LI LU NL SE**

1. Eine Verbindung der Formel

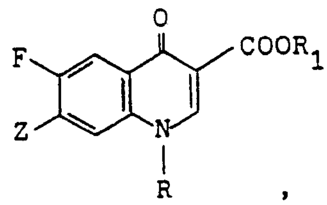

,

worin $R_1$ Wasserstoff oder eine Carboxyschutzgruppe ist; R eine Phenylgruppe der Formel:

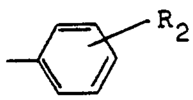

ist, wobei $R_2$ eine oder mehrere Gruppen bedeutet, die aus der Gruppe ausgewählt ist bzw. sind, welche aus Wasserstoff, Halogen, Methylendioxy, $C_1$—$C_6$-Alkyl und einer Gruppe der Formel:

$$—OR_3,$$

wobei $R_3$ Wasserstoff oder $C_1$—$C_6$-Alkyl ist, besteht; und Z einen, heterocyclischen Ring mit der Struktur:

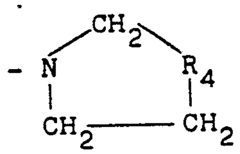

,

wobei $R_4$ $CH_2$, $(CH_2)_2$ oder $(CH_2)_nNH$— ist, wobei n 1 oder 2 ist, und substituierte Derivate davon symbolisiert, wobei die genannten Substituenten aus einem oder mehreren von $C_1$—$C_4$-Alkyl- und —$NR_5R_6$-Resten ausgewählt sind, wobei $R_5$ und $R_6$ unabhängig voneinander aus Wasserstoff, $C_1$—$C_4$-Alkyl und Alkanoyl ausgewählt sind; und pharmazeutisch annehmbare Salze davon.

2. Eine Verbindung der Formel:

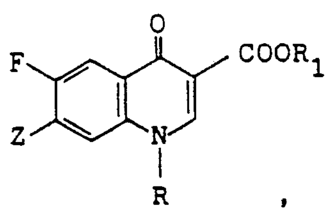

,

worin Z Piperazinyl, $C_1$—$C_6$-Alkyl-substituiertes Piperazinyl oder Amino-substituiertes Pyrrolidinyl ist, R Phenyl oder substituiertes Phenyl ist, wobei der Substituent an der Phenylgruppe einer oder mehrere eines

$C_1$—$C_6$-Alkyl-, Halogen-, Methylendioxy- und Hydroxyrestes sind, und $R_1$ Wasserstoff oder eine Carboxyschutzgruppe ist.

3. Eine Verbindung wie in Anspruch 2 definiert, worin R Phenyl ist, Z Piperazinyl ist, und $R_1$ Wasserstoff ist.

4. Eine Verbindung wie in Anspruch 2 definiert, worin R Phenyl ist, Z 4-Methylpiperazinyl ist, und $R_1$ Wasserstoff ist.

5. Eine Verbindung wie in Anspruch 2 definiert, worin R p-Fluorphenyl ist, Z Piperazinyl ist, und $R_1$ Wasserstoff ist.

6. Eine Verbindung wie in Anspruch 2 definiert, worin R p-Fluorphenyl ist, Z 4-Methylpiperazinyl ist, und $R_1$ Wasserstoff ist.

7. Eine Verbindung wie in Anspruch 2 definiert, worin Z Piperazinyl ist, R 2,4-Difluorphenyl ist, und $R_1$ Wasserstoff ist.

8. Eine Verbindung wie in Anspruch 2 angeführt, worin R Fluorphenyl ist, Z Methylpiperazinyl ist, und $R_1$ Wasserstoff ist.

9. Eine Verbindung wie in Anspruch 2 angeführt, worin R p-Fluorphenyl ist, Z 3-Amino-1-pyrrolidinyl ist, und $R_1$ Wasserstoff ist.

10. Eine Verbindung wie in Anspruch 2 definiert, worin Z Piperazinyl ist, und R Difluorphenyl ist.

11. Eine Verbindung wie in Anspruch 2 definiert, worin Z Methylpiperazinyl ist, und R Difluorphenyl ist.

12. Eine Verbindung wie in Anspruch 2 definiert, worin Z Aminopyrrolidinyl ist, und R 2,4-Difluorphenyl ist.

13. Eine Verbindung wie in Anspruch 2 definiert, wobei Z 3-Methylpiperazinyl ist, R 2,4-Difluorphenyl ist, und $R_1$ Wasserstoff ist.

14. Eine Zusammensetzung mit antibakterieller Wirksamkeit in pharmazeutischer Dosierungsform, enthaltend ein Verdünnungsmittel und eine wie in Anspruch 1 definierte Verbindung.

15. Eine Zusammensetzung mit antibakterieller Wirksamkeit in pharmazeutischer Dosierungsform, enthaltend ein Verdünnungsmittel und eine in Anspruch 2 definierte Verbindung.

16. Eine Zusammensetzung wie in Anspruch 15 definiert, worin Z Piperazinyl ist, R p-Fluorphenyl ist, und $R_1$ Wasserstoff ist.

17. Eine Zusammensetzung wie in Anspruch 15 definiert, worin Z 4-Methylpiperazinyl ist, R p-Fluorphenyl ist, und $R_1$ Wasserstoff ist.

18. Eine Zusammensetzung wie in Anspruch 15 definiert, worin Z 3-Methylpiperazinyl ist, R 2,4-Difluorphenyl ist, und $R_1$ Wasserstoff ist.

19. Verwendung einer Verbindung nach Anspruch 1 zur Hertstellung eines Arzneimittels zur Behandlung einer bakteriellen Infektion in einem Patienten.

20. Verwendung einer Verbindung nach Anspruch 2 zur Herstellung eines Arzneimittels zur Behandlung einer bakteriellen Infektion in einem Patienten.

21. Die wie in Anspruch 20 definierte Verwendung einer Verbindung, worin Z Piperazinyl ist, R p-Fluorphenyl ist, und $R_1$ Wasserstoff ist.

22. Die wie in Anspruch 20 definierte Verwendung einer Verbindung, worin Z 4-Methylpiperazinyl ist, R p-Fluorphenyl ist, und $R_1$ Wasserstoff ist.

23. Die wie in Anspruch 20 definierte Verwendung einer Verbindung, worin Z 3-Methylpiperazinyl ist, R 2,4-Difluorphenyl ist, und $R_1$ Wasserstoff ist.

24. Ein Verfahren zur Herstellung von 1,4-Dihydro-4-oxochinolin-3-carbonsäure, umfassend das Umsetzen der Verbindung der Formel:

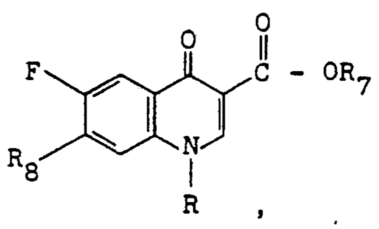

,

worin $R_7$ eine Alkylgruppe mit 1 bis 10 Kohlenstoffatomen ist, $R_8$ eine austretende Gruppe ist, und R eine Phenylgruppe der Formel:

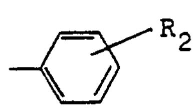

ist, worin $R_2$ eine oder mehrere Gruppen bedeutet, die aus der aus Wasserstoff, Halogen, Methylendioxy, $C_1$—$C_6$-Alkyl und einer Gruppe der Formel:

$$—OR_3,$$

wobei $R_3$ Wasserstoff oder $C_1$—$C_6$-Alkyl ist, bestehenden Gruppe ausgewählt ist bzw. sind; mit einer Verbindung der Formel ZH, worin Z einen heterocyclischen Ring der Struktur:

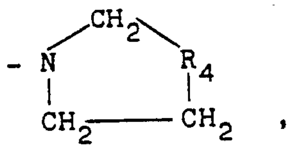

worin $R_4$ $CH_2$, $(CH_2)_2$ oder $(CH_2)_nNH$— ist, wobei n 1 oder 2 ist, und substituierte Derivate davon symbolisiert, wobei die genannten Substituenten aus einer oder mehreren $C_1$—$C_4$-Alkyl- und —$NR_5R_6$-Gruppen ausgewählt sind, wobei $R_5$ und $R_6$ unabhängig voneinander aus Wasserstoff, $C_1$—$C_4$-Alkyl und Alkanoyl ausgewählt sind.

**Patentansprüche für den Vertragsstaat: AT**

1. Ein Verfahren zur Herstellung einer Verbindung der Formel:

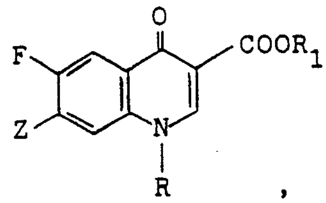

worin $R_1$ Wasserstoff oder eine Carboxyschutzgruppe ist; R eine Phenylgruppe der Formel:

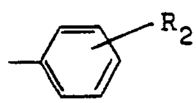

ist, wobei $R_2$ eine oder mehrere Gruppen bedeutet, die aus der Gruppe ausgewählt ist bzw. sind, welche aus Wasserstoff, Halogen, Methylendioxy, $C_1$—$C_6$-Alkyl und einer Gruppe der Formel:

$$—OR_3,$$

wobei $R_3$ Wasserstoff oder $C_1$—$C_6$-Alkyl ist, besteht; und Z einen heterocyclischen Ring mit der Struktur:

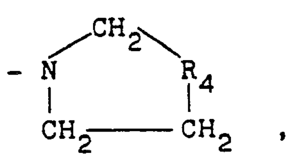

wobei $R_4$ $CH_2$, $(CH_2)_2$ oder $(CH_2)_nNH$— ist, wobei n 1 oder 2 ist, und substituierte Derivate davon symbolisiert, wobei die genannten Substituenten aus einem oder mehreren von $C_1$—$C_4$-Alkyl- und —$NR_5R_6$-Resten ausgewählt sind, wobei $R_5$ und $R_6$ unabhängig voneinander aus Wasserstoff, $C_1$—$C_4$-Alkyl und Alkanoyl ausgewählt sind; und pharmazeutisch annehmbare Salze davon, umfassend:

a) das Umsetzen von 2,4-Dichlor-5-fluor-acetophenon (1) mit Dialkoxycarbonat (2), worin $R_6$ ein $C_1$—$C_{10}$-Alkyl ist, zur Gewinnung eines entsprechenden β-Ketoesters (3):

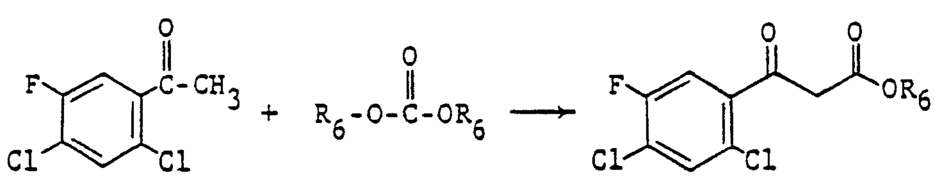

(1) (2) (3)

b) das Behandeln des β-Ketoesters (3) zuerst mit einem Orthoameisensäuretrialkylester (4), worin $R_7$

ein $C_1$—$C_{10}$-Alkyl ist, und dann mit Anilin $NH_2R$, worin R wie oben definiert ist, zur Gewinnung eines Enaminoketoesters (6):

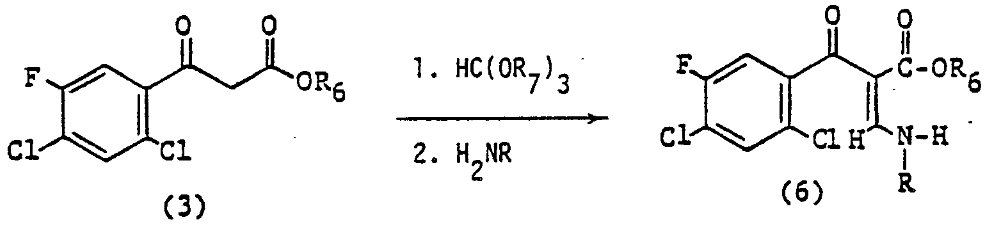


(3) (6)

c) das Cyclisieren des Enaminoketoesters (6) zu einem entsprechenden Chinolin-3-carbonsäureester (7)

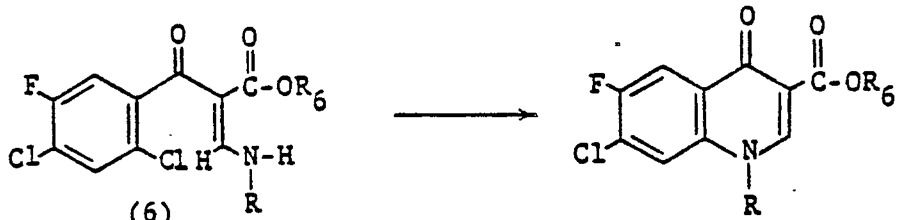

(6) (7)

d) das Hydrolysieren des Esters (7) zu seiner entsprechenden freien 3-Carbonsäure,

e) das Umsetzen der genannten freien Säure aus (d) mit ZH (8), worin Z die oben definiert ist, zur Gewinnung der Verbindung I, worin $R_1$ Wasserstoff ist, und gegebenenfalls das Verestern derselben zur Gewinnung einer Verbindung I, worin $R_1$ eine von Wasserstoff verschieden Bedeutung hat.

2. Ein Verfahren nach Anspruch 1, worin Z Piperazinyl, $C_1$—$C_6$-Alkyl-substituiertes Piperazinyl oder Amino-substituiertes Pyrrolidinyl ist, R Phenyl oder substituiertes Phenyl ist, wobei der Substituent an der Phenylgruppe einer oder mehrere eines $C_1$—$C_6$-Alkyl-, Halogen-, Methylendioxy- und Hydroxyrestes sind, und $R_1$ Wasserstoff oder eine Carboxyschutzgruppe ist.

3. Ein Verfahren wie in Anspruch 2 definiert, worin R Phenyl ist, Z Piperazinyl ist, und $R_1$ Wasserstoff ist.

4. Ein Verfahren wie in Ansruch 2 definiert, worin R Phenyl ist, Z 4-Methylpiperazinyl ist, und $R_1$ Wasserstoff ist.

5. Ein Verfahren wie in Anspruch 2 definiert, worin R p-Fluorphenyl ist, Z Piperazinyl ist, und $R_1$ Wasserstoff ist.

6. Ein Verfahren wie in Anspruch 2 definiert, worin R p-Fluorphenyl ist, Z 4-Methylpiperazinyl ist, und $R_1$ Wasserstoff ist.

7. Ein Verfahren wie in Anspruch 2 definiert, worin Z Piperazinyl ist, R 2,4-Difluorphenyl ist, und $R_1$ Wasserstoff ist.

8. Ein Verfahren wie in Anspruch 2 angeführt, worin R Fluorphenyl ist, Z Methylpiperazinyl ist, und $R_1$ Wasserstoff ist.

9. Ein Verfahren wie in Anspruch 2 angeführt, worin R p-Fluorphenyl ist, Z 3-Amino-1-pyrrolidinyl ist, und $R_1$ Wasserstoff ist.

10. Ein Verfahren wie in Anspruch 2 definiert, worin Z Piperazinyl ist, und R Difluorphenyl ist.

11. Ein Verfahren wie in Anspruch 2 definiert, worin Z Methylpiperazinyl ist, und R Difluorphenyl ist.

12. Ein Verfahren wie in Anspruch 2 definiert, worin Z Aminopyrrolidinyl ist, und R 2,4-Difluorphenyl ist.

13. Eine Verfahren wie in Anspruch 2 definiert, wobei Z 3-Methylpiperazinyl ist, R 2,4-Difluorphenyl ist, und $R_1$ Wasserstoff ist.

14. Ein Verfahren zur Herstellung einer Zusammensetzung mit antibakterieller Wirksamkeit, umfassend das Mischen eines Verdünnigsmittels mit einer nach einem Verfahren gemäß Anspruch 1 erhaltenen Verbindung.

15. Ein Verfahren zur Herstellung einer Zusammensetzung mit antibaktrieller Wirksamkeit, umfassend das Mischen eines Verdünnungsmittels mit einer nach dem Verfahren gemäß Anspruch 2 hergestellten Verbindung.

16. Ein Verfahren wie in Anspruch 15 definiert, worin in der genannten Verbindung Z Piperazinyl ist, R p-Fluorphenyl ist, und $R_1$ Wasserstoff ist.

17. Ein Verfahren wie in Anspruch 15 definiert, worin in der genannten Verbindung Z 4-Methylpiperazinyl ist, R p-Fluorphenyl ist, und $R_1$ Wasserstoff ist.

18. Ein Verfahren wie in Anspruch 15 definiert, worin in der genannten Verbindung Z 3-Methylpiperazinyl ist, R 2,4-Difluorphenyl ist, und $R_1$ Wasserstoff ist.

**Revendications pour les Etats contractants: BE CH DE FR GB IT LI LU NL SE**

1. Un composé ayant la formule suivante:

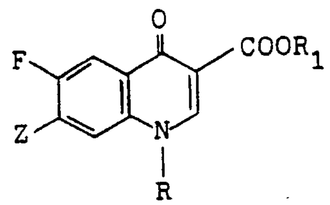

dans laquelle $R_1$ est l'hydrogène ou un groupe protecteur du groupe carboxy; R est un groupe phényle ayant la formule:

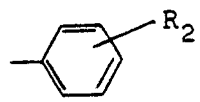

dans laquelle $R_2$ est un ou plusieurs groupes choisis dans le groupe comprenant l'hydrogène, les halogènes, méthylènedioxy, alkyle en $C_1$ à $C_6$, et un groupe ayant la formule:

$$—OR_3$$

dans laquelle $R_3$ est l'hydrogène ou un alkyle en $C_1$ à $C_6$; et Z est un noyau hétérocyclique ayant la structure:

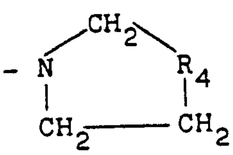

dans laquelle $R_4$ est $CH_2$, $(CH_2)_2$ ou $(CH_2)_nNH—$ où n est 1 ou 2, et les dérivés substitués, dans lesquels lesdits substituants sont choisis parmi un ou plusieurs alkyles en $C_1$ à $C_4$ et $—NR_5R_6$ dans lequel $R_5$ et $R_6$ sont choisis indépendamment parmi l'hydrogène, les alkyles et alcanoyles en $C_1$ à $C_4$; et les sels pharmaceutiquement acceptables de ce composé.

2. Un composé ayant la formule:

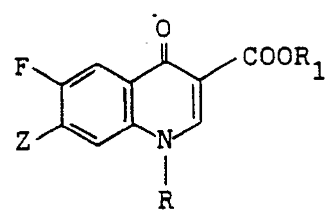

dans laquelle Z est pipérazinyle, pipérazinyle $C_1$—$C_6$ alkyl-substitué, ou pyrrolidinyle amino-substitué, R est phényle ou phényle substitué dans lequel le substituant sur le groupe phényle est un ou plusieurs alkyles en $C_1$—$C_6$, halo, méthylènedioxy et hydroxy et $R_1$ est l'hydrogène ou un groupe protecteur du groupe carboxy.

3. Un composé tel que défini dans la revendication 2, dans laquelle R est phényle, Z est pipérazinyle et $R_1$ est l'hydrogène.

4. Un composé comme défini dans la revendication 2, dans laquelle R est phényle, Z est 4-méthyl-pipérazinyle et $R_1$ est l'hydrogène.

5. Un composé comme défini dans la revendication 2, dans laquelle R est p-fluorophényle, Z est pipérazinyle et $R_1$ est l'hydrogène.

6. Un composé comme défini dans la revendication 2, dans laquelle R est p-fluorophényle, Z est 4-méthylpipérazinyle et $R_1$ est l'hydrogène.

7. Un composé comme défini dans la revendication 2, dans laquelle Z est pipérazinyle, R est 2,4-difluorophényle et $R_1$ est l'hydrogène.

8. Un composé tel que récité dans la revendication 2, dans laquelle R est fluorophényle, Z est méthylpipérazinyle, et $R_1$ est l'hydrogène.

9. Un composé tel que récité dans la revendication 2, dans laquelle R est p-fluorophényle, Z est 3-amino-1-pyrrolidinyle et $R_1$ est l'hydrogène.

10. Un composé tel que défini dans la revendication 2, dans laquelle Z est pipérazinyle et R est difluorophényle.

11. Un composé tel que défini dans la revendication 2, dans laquelle Z est méthylpipérazinyle et R est difluorophényle.

12. Un composé tel que défini dans la revendication 2, dans laquelle Z est aminopyrrolidinyle et R est 2,4-difluorophényle.

13. Un composé tel que défini dans la revendication 2, dans laquelle Z est 3-méthylpipérazinyle, R est 2,4-difluorophényle et $R_1$ est l'hydrogène.

14. Une composition ayant une activté antibactérienne sous forme de dosage pharmaceutique contenant un diluant et un composé tel que défini dans la revendication 1.

15. Une composition ayant une activté antibactérienne sous forme de dosage pharmaceutique contenant un diluant et un composé défini dans la revendication 2.

16. Une composition telle que définie dans la revendication 15, dans laquelle Z est pipérazinyle, R est p-fluorophényle et $R_1$ est l'hydrogène.

17. Une composition telle que définie dans la revendication 15, dans laquelle Z est 4-méthylpipérazinyle, R est p-fluorophényle et $R_1$ est l'hydrogène.

18. Une composition telle que définie dans la revendication 15, dans laquelle Z est 3-méthylpipérazinyle, R est 2,4-difluorophényle et $R_1$ est l'hydrogène.

19. Utilisation d'un composé selon la revendication 1 pour la fabrication d'un médicament pour le traitement d'une infection bactérienne chez un patient.

20. Utilisation d'un composé selon la revendication 2 pour fabriquer un médicament pour le traitement d'une infection bactérienne chez un patient.

21. Utilisation telle que décrite dans la revendication 20 d'un composé dans lequel Z est pipérazinyle, R est p-fluorophényle et $R_1$ est l'hydrogène.

22. Utilisation telle que définie dans la revendication 20 d'un composé dans lequel Z est 4-méthylpipérazinyl, R est p-fluorophényle et $R_1$ est l'hydrogène.

23. Utilisation telle que définie dans la revendication 20 d'un composé dans lequel Z est 3-méthylpipérazinyle, R est 2,4-difluorophényle et $R_1$ est l'hydrogène.

24. Un procédé de production des acides 1,4-dihydro-4-oxo-quinoléine-3-carboxyliques comprenant la réaction d'un composé de formule:

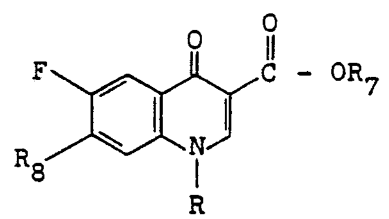

dans laquelle $R_7$ est un groupe alkyle de 1 à 10 atomes de carbone, $R_8$ est un groupe qui s'élimine et R est un groupe phényle de formule:

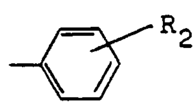

dans laquelle $R_2$ est un ou plusieurs groupes choisis dans le groupe comprenant l'hydrogène, les halogènes, méthylènedioxy, alkyle en $C_1$ à $C_6$ et un groupe ayant la formule:

$$—OR_3$$

dans laquelle $R_3$ est l'hydrogène ou un alkyle en $C_1$ à $C_6$; avec un composé de formule ZH dans laquelle Z est un noyau hétérocyclique ayant la structure suivante:

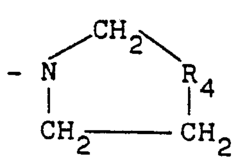

dans laquelle $R_4$ est $CH_2$, $(CH_2)_2$ ou $(CH_2)_nN—$ où n est 0, 1 ou 2 et les dérivés substitués, dans lesquels les substituants sont choisis parmi un ou plusieurs des radicaux alkyles en $C_1$ à $C_4$ et $—NR_5R_6$ dans lequel $R_5$ et $R_6$ sont choisis indépendamment parmi l'hydrogène, les groupes alkyles et alcanoyles en $C_1$ à $C_4$.

**Revendications pour l'Etat contractant: AT**

1. Un procédé pour préparer un composé ayant la formule:

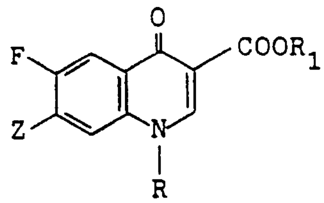

dans laquelle $R_1$ est l'hydrogène ou un groupe protecteur du groupe carboxy; R est un groupe phényle ayant la formule:

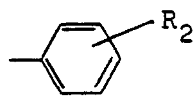

dans laquelle $R_2$ est un ou plusieurs groupes choisis dans le groupe comprenant l'hydrogène, les halogènes, méthylènedioxy, alkyle en $C_1$ à $C_6$, et un groupe ayant la formule:

$$—OR_3$$

dans laquelle $R_3$ est l'hydrogène ou un alkyle en $C_1$ à $C_6$; et Z est un noyau hétérocyclique ayant la structure:

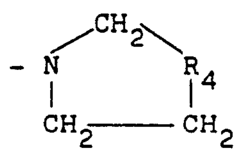

dans laquelle $R_4$ est $CH_2$, $(CH_2)_2$ ou $(CH_2)_nNH—$, dans lequel n est 1 ou 2, et les dérivés substitués, dans lesquels lesdits substituants sont choisis parmi un ou plusieurs des groupes alkyles en $C_1$ à $C_4$ et $—NR_5R_6$ dans lequel $R_5$ et $R_6$ sont choisis indépendamment parmi l'hydrogène, alkyle et alcanoyle en $C_1$ à $C_4$; et les sels pharmaceutiquement acceptables, comprenant:

a) la réaction de la 2,4-dichloro-5-fluoro-acétophénone (1) avec le dialcoxycarbonate (2) dans lequel $R_6$ est un alkyle en $C_1—C_{10}$, pour obtenir un β-cétoester correspondant (3):

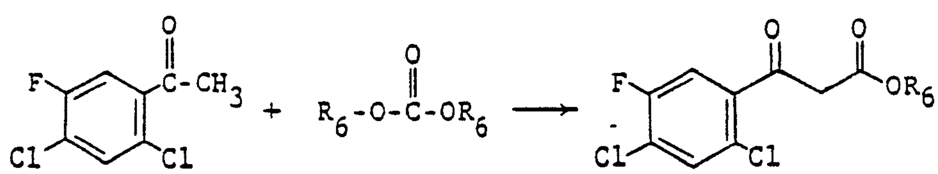

(1) (2) (3)

b) le traitement du β-cétoester (3) en premier lieu avec un orthoformiate de trialkyle (4), dans lequel $R_7$ est un alkyle en $C_1—C_{10}$, et ensuite avec un aniline $NH_2R$, dans laquelle R est comme défini précédemment pour obtenir un ènaminocétoester (6):

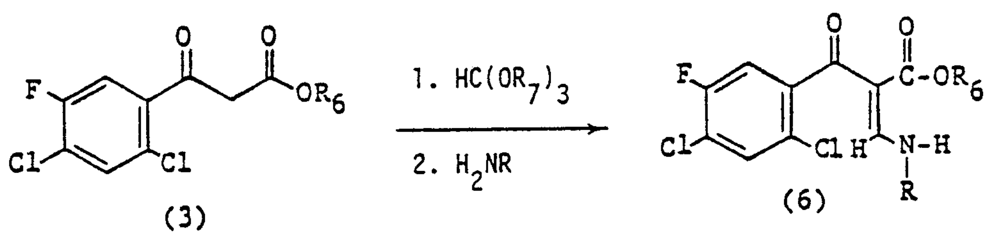


(3) (6)

c) la cyclisation de l'ènaminocétoester (6) en ester d'acide quinoléine-3-carboxylique (7) correspondant

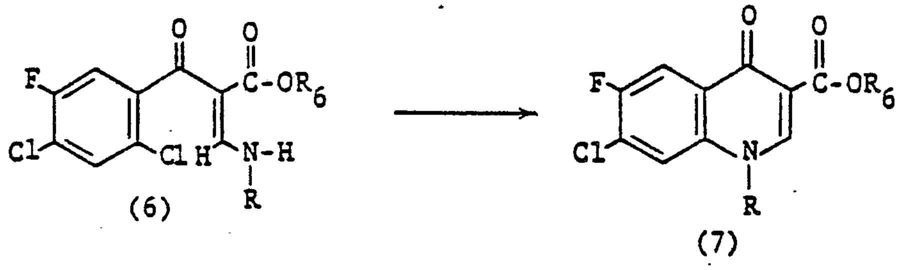

(6) (7)

d) l'hydrolyse de l'ester (7) en son acide 3-carboxylqiue libre correspondant,

e) la réaction de cet acide libre provenant de (d) avec ZH (8) dans lequel Z est comme défini précédemment pour obtenir le composé I dans lequel $R_1$ est l'hydrogène, et éventullement l'estérification de ce dernier pour obtenir un composé I dans lequel $R_1$ est différent de l'hydrogène.

2. Un procédé selon la revendication 1, dans laquelle Z est pipérazinyle, pipérazinyle $C_1$—$C_6$ alkyl-substitué, ou pyrrolidinyle amino substitué, R est phényle ou phényle substitué dans lequel le substituant sur le groupe phényle est un ou plusieurs alkyles en $C_1$—$C_6$, halo, méthylènedioxy et hydroxy et $R_1$ est l'hydrogène ou un groupe protecteur du groupe carboxy.

3. Un procédé tel que défini dans la revendication 2, dans laquelle R est phényle, Z est pipérazinyle et $R_1$ est l'hydrogène.

4. Un procédé tel que comme défini dans la revendication 2, dans laquelle R est phényle, Z est 4-méthylpipérazinyle et $R_1$ est l'hydrogène.

5. Un procédé tel que défini dans la revendication 2, dans laquelle R est p-fluorophényle, Z est pipérazinyle et $R_1$ est l'hydrogène.

6. Un procéde tel que défini dans la revendication 2, dans laquelle R est p-fluorophényle, Z est 4-méthylpipérazinyle et $R_1$ est l'hydrogène.

7. Un procéde tel que défini dans la revendication 2, dans laquelle Z est pipérazinyle, R est 2,4-difluorophényle et $R_1$ est l'hydrogène.

8. Un procédé tel que récité dans la revendication 2, dans laquelle R est fluorophényle, Z est méthylpipérazinyle, et $R_1$ est l'hydrogène.

9. Un procédé tel que récité dans la revendication 2, dans laquelle R est p-fluorophényle, Z est 3-amino-1-pyrrolidinyle et $R_1$ est l'hydrogène.

10. Un procédé tel que défini dans la revendication 2, dans laquelle Z est pipérazinyle et R est difluorophényle.

11. Un procédé tel que défini dans la revendication 2, dans laquelle Z est méthylpipérazinyle et R est difluorophényle.

12. Un procédé tel que défini dans la revendication 2, dans laquelle Z est aminopyrrolidinyle et R est 2,4-difluorophényle.

13. Un procédé tel que défini dans la revendication 2, dans laquelle Z est 3-méthylpipérazinyle, R est 2,4-difluorophényle et $R_1$ est l'hydrogène.

14. Une procédé pour préparer une composition ayant une activité antibactérienne comprenant le mélange d'un diluant avec un composé obtenu par un procédé selon la revendication 1.

15. Une procédé pour préparer une composition ayant une activite antibactérienne comprenant le mélange d'un diluant avec un composé préparé selon le procédé de la revendication 2.

16. Une procédé tel que défini dans la revendication 15, dans laquelle dans ledit composé Z est pipérazinyle, R est p-fluorophényle et $R_1$ est l'hydrogène.

17. Une procédé tel que défini dans la revendication 15, dans laquelle dans ledit composé Z est 4-méthylpipérazinyle, R est p-fluorophényle et $R_1$ est l'hydrogène.

18. Une procédé tel que défini dans la revendication 15, dans laquelle dans ledit composé Z est 3-méthylpipérazinyle, R est 2,4-difluorophényle et $R_1$ est l'hydrogène.